# EUROPEAN PATENT APPLICATION

(11) **EP 3 381 888 A1**
(43) Date of publication of application: **03.10.2018**
(21) Application number: 15909129.7
(22) Date of filing: 17.12.2015
(51) Int. Cl.: C07C 227/18

(54) **METHOD OF CO-PRODUCING LONG CHAIN AMINO ACID AND DIBASIC ACID**

(30) Priority: 27.11.2015 CN 201510848578
(71) Applicant: Dasmart Environmental Science and Technologies (Beijing) Co., Ltd, Beijing 100080 (CN)
(72) Inventor: HU, Songzhou, Princeton, New Jersey 08540 (US)
(74) Representative: J A Kemp
(86) International application number: PCT/CN2015/097705
(87) International publication number: WO 2017/088218

(57) **Abstract**

A method of co-producing a long-chain terminal amino acid and a dibasic acid, comprising the following steps: (a) reacting a keto acid derivative with hydroxyl amine in a solvent or performing an ammoxidation oximation reaction to generate a oximino acid derivative; (b) performing a Beckmann rearrangement reaction on the produced oximino acid derivative to produce a mixed amide derivative; and (c) hydrolyzing the obtained mixed amide derivative to obtain a long chain terminal amino acid and long chain dibasic acid.

## Description

### Field of the Invention

The present invention relates to a method of producing a long-chain nylon monomer, particularly to a method of co-producing a long-chain terminal amino acid and a long-chain dibasic acid.

### Background of the Invention

Long-chain saturated fatty terminal amino acids, lactams and dibasic acids are important monomers in industrial production of long-chain nylon engineering plastics. Nylon is a kind of high molecular polymer containing an amide group on the main chain of the molecule. Nylon is the most consumed engineering plastic and of the largest variety, and is used most widely.

The long-chain nylon with a unique molecular structure has good overall performance, strength higher than metals, low water absorption, good oil resistance, low temperature resistance, wear resistance, chemical resistance, flame retardancy, self-lubrication and small friction coefficient and is easy to process. The long-chain nylon can be processed into a variety of plastic products, can also be drawn into fibers, and can be made into films, coatings and thermal adhesives widely used in the fields of automobiles, electronics and electrics, machinery, communications, petrochemicals and aerospace.

Nylon 9, nylon 11 and nylon 12 are industrially produced using long-chain amino acids or lactams as monomers.

Important nylon 610, nylon 612, nylon 1010 and nylon 1212 are industrially produced using long-chain dibasic acids and diamines as raw materials.

In the existing industrial production technologies, monomeric 9-aminononanoic acid of nylon 9 is obtained from a series of chemical reactions of oleic acid or oleonitrile (detailed description can be found in J. Am Oil Chemist's Soc., 1975, Vol. 52, No. 11, pp 473-477). First of all, oleic acid is oxidized with ozone and hydrogenated to prepare a methyl formylcaprylate intermediate. 9-methyl aminononanoate is generated after reductive aminolysis, hydrolyzed, and purified to obtain a 9-aminononanoic acid monomer. The total yield is about 35%.

As a nylon 11 monomer, 11-aminoundecanoic acid is obtained from castor oil as a raw material through methanation, high-temperature pyrolysis, anhydrous hydrogen bromide radical addition and final aminolysis (the detailed process is described in the document A. Chauvel & G. Lefebvre, Petrochemical Processes 2: Major Oxygenated, Chlorinated and Nitrated Derivatives, pages 274-278). The total yield does not exceed 55%.

As a nylon 12 monomer, laurolactam is obtained, starting from butadiene industrially, through a series of reactions including catalytic trimerization, hydrogenation reduction, oxidation to cyclododecanone, oximation and Beckmann rearrangement (the detailed process is described in the document A. Chauvel & G. Lefebvre Petrochemical Processes 2: Major Oxygenated, Chlorinated and Nitrated Derivatives, pages 279-286).

In the industrial production of long-chain dibasic acids, azelaic acid is obtained by complete oxidation of oleic acid. Qualified sebacic acid is obtained by decomposing a ricinoleic acid with a strong base at a high temperature (200°C-250°C) to prepare a crude product and refining the crude product by a series of purification.

For the important dodecanedioic acid, two quite different routes are adopted industrially. One is chemical synthesis, starting from butadiene, through catalytic trimerization, hydrogenation reduction, oxidation to an alcohol and a ketone and full oxidation with nitric acid. However, the dodecanedioic acid is produced more by oxidizing high-purity n-dodecane or laurate with a biological fermentation method at present.

When these monomers are produced using chemical synthesis methods, the existing methods have the main problems of low reaction yield (only 35% for 9-aminoacidonanoic acid, only 55% for 11-aminoundecanoic acid, only 80% for azelaic acid), violent reaction conditions and very low insecurity. For example, ozonation is used for producing the 9-aminononanoic acid, high-temperature pyrolysis is used for producing the 11-aminoundecanoic acid, anhydrous anaerobic trimerization is used for producing the laurolactam and the dodecanedioic acid, and high-temperature base decomposition is used for producing the sebacic acid.

The bio-fermentation for producing the dodecanedioic acid and other long-chain dibasic acids is mild in reaction condition, but the crude product contains mass biomass and degraded short-chain diacid impurities. In order to obtain a qualified product suitable for producing nylon, the crude product must undergo a large amount of complicated refining. A variety of refining methods are described in a number of patent documents, and the detailed processes can be found in U.S. patents U.S.6,218,574 and U.S.8,729,298 and Chinese patents CN 104591998A, CN 102476990A, CN 102329224A, CN 103497100A, CN 102795989A, CN 104447274A, CN 104447280A, CN 104496793A, CN 104529741A and CN 104529747A.

### Summary of the Invention

Aiming at the shortcomings of the prior art, the present invention provides a method for co-producing a long-chain amino acid and a dibasic acid. Compared with the existing industrial production technology, the production method provided by the present invention is mild in reaction condition and high in overall yield, greatly improves the product quality, and is suitable for industrialized production.

In the present invention, a long-chain keto acid derivative (I) is used as a raw material to co-produce a long-chain terminal amino acid (V) and a dibasic acid (IV) according to the following steps:
(1) performing an oximation reaction or an ammoxidation oximation reaction on a keto acid derivative (I) and hydroxyl amine in a solvent to generate a oximino acid derivative (II);
(2) performing Beckmann rearrangement on the oximino acid derivative under the catalysis of a catalyst to generate a mixed amide derivative including compounds shown by formula (IIIa) and formula (IIIb); and
(3) hydrolyzing the Beckmann rearrangement product, i.e., the mixed amide derivative including compounds shown by formula (IIIa) and formula (IIIb) to obtain the long-chain amino acid (V) and the dibasic acid (IV). The hydrolysis reaction also produces a short-chain primary amine and a short-chain alkanoic acid.

The above reaction process is as follows:

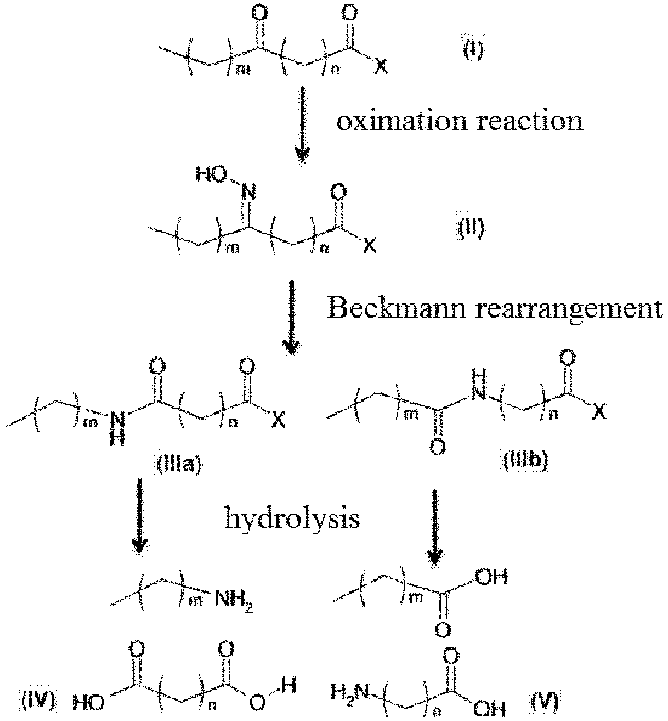

In the reaction equation, X is OR or NR1R2; OR is a monohydric alcohol or polyhydric alcohol of C1-C8, such as ethylene glycol, propylene glycol, butanediol or glycerin, etc. R1 and R2 are respectively hydrogen and C1-C8 alkyl. m is an integer from 0 to 10. n is an integer from 6 to 20. Preferably, X is OR, i.e., a keto ester.

It is noteworthy that when m=5 and n=10, the raw material is 12-ketostearic acid derivative. The production method disclosed by the present invention can produce a nylon 11 monomer, i.e.,11-aminoundecanoic acid, and also co-produces a very important dodecanedioic acid for producing nylon 612 and nylon 1212.

When m=7 and n=8, the raw material is 10-ketostearic acid derivative. After the process of the present invention, the obtained product is 9-aminononanoic acid, i.e., a nylon 9 monomer. Sebacic acid is also co-produced for producing nylon 610 and nylon 1010.

When m=5 and n=12, the raw material is 14-ketoarachidic acid derivative. The product obtained by the above reaction is 13-aminotridecanoic acid, which is used for synthesizing nylon 13. Tetradecanoic diacid is produced at the same time.

In the forming step of the oximino acid derivative (II), a reaction or an ammoxidation oximation reaction is performed on the keto acid derivative (I) and an aqueous solution of hydroxyl amine in an organic solvent to generate the oximino acid derivative (II). The organic solvent may be a water-soluble or water-insoluble organic solvent. The solvent for the oximation reaction is selected to ensure that both the keto acid derivative (I) and the oximino acid derivative (II) are easily dissolved into the solvent, and does not react with the reactant, the product and the hydroxyl amine. For example, aldehydes and ketones cannot be used for the oximation reaction because they react with the hydroxyl amine themselves. Nitrile solvents also react with the hydroxyl amine and are thus not suitable. Ammonia solvents react with ketone to generate Schiff bases, and are also not suitable. Alcoholic solvents, which can undergo an ester shift reaction with the keto acid derivative (I), are preferably not adopted.

For the Beckmann rearrangement reaction, the solvent is required to have better solubility for the oximino acid derivative (II) and the amide derivative (III), and to dissolve the Beckmann rearrangement catalyst without reacting with the catalyst.

The solvents required for the oximation and the Beckmann rearrangement reaction must be stable and easy to recover. The oxidation reaction and the Beckmann rearrangement reaction may adopt different solvents to meet the requirements thereof. However, the preferred solvent should meet the requirements of the two reactions at the same time, thus reducing the use and recovery of the solvent. The preferred organic solvent is preferably insoluble in water, so that the product is conveniently separated after the oximation and the rearrangement reaction. The quantity of the solvent does not need to be particularly limited, because the solvent in the present invention only functions to dilute and disperse the reactants.

The solvent that can meet the above requirements includes ester, alkane, aromatic hydrocarbon, ether and other inert solvent. For example, the preferred solvent is butyl acetate, ethyl acetate, benzene, toluene, xylene, cumene, anisole, diethyl ether, isopropyl ether, butyl ether, methyl tert-butyl ether, ethyl tert-butyl ether, methyl tetrahydrozonam, petroleum ether, cyclohexane, dichloroethane, dichloromethane, chloroform, carbon tetrachloride or benzotrifluoride.

These solvents may be used alone or used as a mixture of two or more.

The temperature of the oximation reaction is 0°C to 100°C, but the oximation reaction can also be carried out at a higher temperature under increased pressure. This reaction is preferably carried out at 0°C to 100°C under normal pressure. If the temperature is too low, the reaction is very slow, and the reaction time is long. The preferred reaction temperature is 60°C to 80°C.

The oximation reaction can be carried out in air, or carried out under the protection of an inert gas such as nitrogen, argon or helium.

The oxidation reaction time is related to the temperature, and is usually 0.5 hour to 24 hours. The preferred time is 1 to 6 hours, and the reaction temperature is controlled between 0 and 100°C. If the reaction time is too short, the quantity of residual keto acid derivative is too large, thus reducing the reaction yield. The residual keto acid derivative can be recovered at post treatment, but additional recovery equipment is required. The long reaction time can reduce the residual quantity of the keto acid derivative, but the reaction equipment must be enlarged.

The oximation reactor may be a common reactor, such as a batch reactor, a semi-continuous reactor, a tubular reactor, or a flow reactor. A continuous stirred tank reactor (CSTR) is preferred. If the CSTR is adopted, the aqueous solution of the hydroxyl amine and the organic solvent for the keto acid derivative (I) can be simultaneously fed to a first reactor, and then flow to the remaining reactors to complete the reaction.

If the aqueous solution of a hydroxyl amine salt such as sulfate or hydrochloride is used in the oximation reaction, the pH value of the reaction should be adjusted to 3-14 using an alkaline substance, preferably aqueous ammonia, to ensure the reaction. After the reaction, the aqueous solution can be used for recovering ammonium salts, such as ammonium sulfate. The oximation reaction may also be ammoxidation oximation reaction, which may be carried out according to a conventional disclosed process, that is, oxidizing ammonia with hydrogen peroxide and carrying out oximation with the keto acid derivative (I).

The molar ratio of the keto acid derivative (I) to the hydroxyl amine can be controlled to 0.1-10.0, preferably 1.0-2.0, to ensure that the keto acid derivative (I) is converted into theoximino acid derivative (II).

After the reaction, the oximino acid derivative (II) is dissolved into the organic phase. The aqueous phase is separated and the organic phase is washed. Although water has little solubility in the organic phase, it will destroy the activity of the Beckmann rearrangement catalyst, so it must be removed. In order to remove a small amount of water, a drying agent or a dehydrating agent can be used. The preferred method is to remove water by evaporating a small amount of solvent. The evaporated solvent can be directly used for the oximation reaction without being dried. The remaining non-aqueous oximino acid derivative solution can be directly used for the Beckmann rearrangement reaction.

The dehydrated oximino acid derivative (II) is heated to generate the amide derivative (III) through Beckmann rearrangement under the action of a catalyst. The catalyst used is sulfuric acid or an compound containing acidic active halogen or a mixture of Lewis acid and compound containing the acidic active halogen. The acidic active halogen compound can be used alone to catalyze the Beckmann rearrangement reaction. The combined catalyst containing the Lewis acid can achieve a better reaction effect. The acidic active halogen compound is preferably a chlorine-containing compound.

The appropriate acidic active chlorine compound is any one of thionyl chloride, chlorosulfone, chlorosulfonic acid, various sulfonyl chlorides (methanesulfonyl chloride, benzenesulfonyl chloride, p-toluenesulfonyl chloride), various acyl chlorides (e.g., formyl chloride, acetyl chloride, benzoyl chloride, oxalyl chloride), phosgene, diphosgene, triphosgene and boron trichloride or a mixture of two or more of them in any ratio, or any one of various chlorine-containing phosphorus compounds, such as phosphorus trichloride, phosphorus pentachloride and phosphorus oxychloride or a mixture of two or more of them in any ratio. In addition, an active acidic chloride-containing heterocyclic compound, particularly chloronitrile trimer or phosphazene, can also be used as the catalyst.

The available Lewis acid includes metal halides, e.g., any one of zinc chloride, ferric chloride, cobalt chloride, stannic chloride, aluminum chloride, titanium chloride and boron trichloride, or a mixture of two or more of them in any ratio.

In the above Beckmann rearrangement reaction, the amount of the acidic active chlorine compound is the amount of the catalyst, i.e., not more than 10% of the molar weight of the oximino acid derivative, preferably 0.1-5% of the molar weight of the oximino acid derivative. The amount of the acidic active chlorine compound and the Lewis acid is also the amount of the catalyst, i.e., not more than 10% of the molar weight of the oximino acid derivative, preferably 0.1-5% of the molar weight of the oximino acid derivative. When the rearrangement is catalyzed with sulfuric acid, the amount of the sulfuric acid can conform to the traditional disclosed process.

The molar ratio of the Lewis acid to the acidic active chlorine is 1: 0.01 to 1:100, preferably 1:0.3 to 1:1.5.

The amount of the catalyst, the reaction temperature, the reaction pressure and the reaction time are correlated with each other. At a specific reaction temperature, increasing the amount of the catalyst can shorten the reaction time.

The Beckmann reaction temperature of the oximino acid derivative is not critical, and the reaction can be completed from the normal temperature to the reflux temperature. The reaction can also be completed by heating under increased pressure. If the temperature is too high, the color of the product will be deepened, which is not conducive to post treatment.

The rearrangement reaction can be carried out in air, or carried out under the protection of an inert gas such as nitrogen, argon or helium. The reaction is preferably completed in dry air. The rearrangement reaction is not limited by the pressure, but can be carried out under normal pressure, reduced pressure or increased pressure.

The rearrangement reaction also has no restriction on the reactor. The common reactor or tubular reactor is available. The reaction can be carried out intermittently, semi-continuously or continuously.

After the reaction, the active catalyst can be quenched, or continuously reused after the product is separated. The quenching reaction if necessary can be completed by adding a small amount of water. The added water can also contain a small amount of acid or alkali, and some inorganic salts can be added.

The amide derivative generated by the Beckmann reaction is a mixture of two amides, (IIIa) and (IIIb), and their molar ratios are almost the same. After the solvent is recovered, the mixture can be purified to obtain the pure amide derivative and then undergo the hydrolysis step, whereas the crude product can also be hydrolyzed directly and the introduced impurities are removed after hydrolysis. In fact, the rearrangement product is very pure if the purity of the hydroxamate (II) is very high.

The amide derivative can be hydrolyzed in an acid. The acid used may be any one of sulfuric acid, hydrochloric acid, hydrobromic acid, hydroiodic acid, nitric acid and the like, or a mixture of two or more of them in any ratio. The amount of the acid used in the hydrolysis reaction and the reaction condition can be in accordance with the traditional disclosed process. During the reaction, an organic solvent such as methanol, ethanol, formic acid, acetic acid or the like may be added in order to increase the solubility of the mixed amide derivative including the compounds shown by formulas (IIIa) and (IIIb).

After the hydrolysis, the almost pure saturated dibasic acid can be separated by cooling crystallization. After the mother solution is neutralized to be neutral, the long-chain amino acid can be separated. The long-chain dibasic acid and amino acid are refined by recrystallization to obtain qualified products.

The long-chain mixed amide derivative including the compounds shown by formula (IIIa) and formula (IIIb) may also be hydrolyzed with an alkali. The amount of the alkali used in the hydrolysis reaction and the reaction condition can be in accordance with the traditional disclosed process. The alkali used may be any one of sodium hydroxide, potassium hydroxide, sodium carbonate, potassium carbonate, barium hydroxide and the like, or a mixture of two or more of them in any ratio. The hydrolyzed solution may be water or a mixed solvent of water and an organic solvent in any ratio. The organic solvent may be any one of methanol, ethanol, isopropanol, tetrahydrofuran, dioxane and the like, or a mixture of two or more of them in any ratio.

The temperature of the hydrolysis reaction is preferably 50°C to 200°C, and the pressure is preferably from normal pressure to self-generated pressure of the hydrolysis temperature, and may also be increased. The hydrolysis reaction is preferably carried out in air or carried out under the protection of an inert gas.

The hydrolysis time is determined by the alkali concentration and the reaction temperature, and is 1 to 24 hours. The preferred time is 2 to 4 hours. If the reaction time is too short, the hydrolysis reaction cannot be completed. If the reaction time is too long, the volume of the reactor increases, thus increasing unnecessary investment.

After the hydrolysis, the organic solvent is removed, and the pH is neutralized with acid to precipitate the long-chain amino acid. The acid used is sulfuric acid, hydrochloric acid, nitric acid, or organic acid such as acetic acid, propionic acid, citric acid, tartaric acid or the like. The inorganic acid is preferred.

After the amino acid is precipitated, the pH value of the mother solution is continuously adjusted to be less than 5, and the long-chain dibasic acid can be precipitated. The product is separated by filtration.

It is worth noting that the long-chain dibasic acid and amino acid prepared by the present invention have very high purity and do not contain any impurities such as other dibasic acids and iminodibasic acids.

### Detailed Description of the Embodiments

The solutions of the present invention are further described in detail below in combination with specific embodiments, but the present invention is not limited to these embodiments.

### Embodiment 1. Co-production of nylon 11 monomer and dodecanedioic acid

94 g of 12-keto methyl stearate is dissolved into 500 mL of toluene, and an aqueous solution of hydroxylamine sulfate (∼8%) containing 13.5 g of hydroxylamine is added. The solution is stirred intensely at 70-85°C, the pH of the system is maintained between 4.5 and 5.0 with aqueous ammonia, and the HPLC analysis after 6 hours shows that the raw material is completely converted into 12-oxime methyl stearate.

After the reaction, standing stratification is performed, and the aqueous phase is discharged. Then, 1.1 g of chloronitrile trimer and 1.5 g of zinc chloride are added. The reaction is carried out at 90-105°C for 2 hours while stirring, until Beckman rearrangement is completed. The reaction is terminated by adding 50 mL of water. The toluene is recovered after the aqueous phase is separated to obtain off-white mixed methyl amidate.

The above solid is heated and dissolved into 500mL of 10% aqueous solution of sodium hydroxide, the solution is placed into an autoclave and incubated at 150°C for 4 hours, and the HPLC analysis shows that the hydrolysis is completed.

500 mL of water is added into the hydrolysate, 2 g of active carbon is added to decolor the hydrolysate at 90°C for 30 minutes, and then the hydrolysate is neutralized to pH 7.5 with dilute sulfuric acid. After the hydrolysate is cooled to room temperature, 55.8 g of 11-aminoundecanoic acid is obtained by filtering, washing and drying. The HPLC analysis shows that the purity of the product is 99.7%.

The mother solution is heated to 85°C and continuously acidified to pH 1 with sulfuric acid to precipitate a large amount of solid. The mother solution is cooled to room temperature, and the solid is filtered, washed three times with distilled water and once with methanol. 62.4 g of dodecanedioic acid is obtained by drying. The HPLC analysis shows that the purity of the product is 99.5%.

### Embodiment 2. Co-production of nylon 9 monomer and decanedioic acid

94 g of 10-keto methyl stearate is dissolved into 500 mL of butyl acetate, and an aqueous solution of hydroxylamine sulfate (∼8% concentration) containing 12.5 g of hydroxylamine is added. The solution is stirred intensely at 70-80°C, and the pH of the system is maintained between 4.5 and 5.0 with aqueous ammonia. The HPLC analysis shows that the raw material is completely converted into 10-oxime methyl stearate.

The aqueous phase is separated. After the organic phase is distilled off with 50 mL of water, 0.8 g of triphosgene and 1.2 g of zinc chloride are added. The reaction is carried out at 90°C for 3 hours under stirring, until the rearrangement reaction is ended. The reaction is terminated by adding 50 mL of water. The solvent is recovered after the aqueous phase is separated. Off-white mixed methyl amidate is obtained.

The above solid is dissolved into 200 mL of acetic acid, then 200 mL of 30% hydrochloric acid is added, and the solution is refluxed for 48 hours till the hydrolysis is completed. 500 mL of water is added for cooling crystallization. 54.6 g of decanedioic acid is obtained by filtering and drying. The HPLC analysis shows that the purity of the product is 99.5%.

The mother solution is evaporated to dryness under reduced pressure. The solid is dissolved into 800 mL of water, and the solution is heated to 80°C. The solution is neutralized with aqueous ammonia to pH 6.5-7.0. Cooling, filtration and drying are performed. 45.6 g of 9-aminononanoic acid is obtained. The HPLC analysis shows that the purity of the product is 99.6%.

### Embodiment 3. Co-production of nylon 13 monomer and tetradecenoic acid

102.2 g of 14-keto methyl arachidate is dissolved into 500 mL of anisole, and an aqueous solution of hydroxylamine sulfate (8% concentration) containing 16.5 g of hydroxylamine is added. The solution is stirred intensely at 75-85°C, and the pH of the system is maintained between 4.5 and 5.0 with aqueous ammonia. The HPLC analysis shows that the raw material is completely converted into 14-oxime methyl arachidate.

The aqueous phase is separated. The organic phase is distilled under reduced pressure to remove 50 mL of water, 1.2 g of p-toluenesulfonyl chloride and 1.5 g of zinc chloride are added, the reaction is carried out for 2 hours at 90-105°C under stirring to complete Beckmann rearrangement reaction, and the reaction is terminated by adding 50 mL of water. The anisole is recovered under reduced pressure after the aqueous phase is separated, and the residue is cooled and solidified to a mixed methyl amidate.

The above solid is heated and dissolved into 700 mL of 8% aqueous solution of sodium hydroxide, and the solution is placed into an autoclave and hydrolyzed at 150°C for 5 hours. The HPLC analysis shows that the hydrolysis is completed.

600 mL of water is added into the hydrolysate, and 2 g of active carbon is added to decolor the hydrolysate at 90°C for 45 minutes. After the carbon is removal by filtration, the solution is neutralized with 30% hydrochloric acid at 80-90°C to pH 7.0-7.5. The solution is cooled to room temperature, and the product is filtered, washed with water and dried to obtain 62.7 g of 13-aminotridecanoic acid. The HPLC analysis shows that the purity of the product is 99.5%.

The mother solution is heated to 85°C and continuously acidified with 30% hydrochloric acid to pH 1 to generate a large amount of solid. The solution is cooled to room temperature, and the solid is filtered, washed with water, washed with methanol and dried to obtain 70.6 g of tetradecanedioic acid. The HPLC analysis shows that the purity of the product is 99.7%.

### Embodiment 4. Ammoxidation oximation reaction of 12-keto methyl stearate

95 g of 12-keto methyl stearate is dissolved into 500 mL of toluene, 20 g of titanium silicalite molecular sieves are added, the temperature is raised to 70°C with stirring, and 50 mL of hydrogen peroxide with a mass fraction of 27.5% and 70 mL of aqueous ammonia with a mass fraction of 25% are simultaneously and uniformly dripped within 2 hours. The reaction is carried out at 75 ° C for 60 minutes under stirring, and then cooling and standing delamination are carried out. The HPLC analysis shows that the raw material is completely converted into 12-oxime methyl stearate. The subsequent operation follows embodiment 1 to obtain 56.5 g of 11-aminoundecanoic acid. The HPLC analysis shows that the purity of the product is 99.5%; 63.7 g of dodecanedioic acid. The HPLC analysis shows that the purity of the product is 99.3%.

### Embodiment 5. Beckmann rearrangement reaction of sulfuric acid catalyzed 12-oxime methyl stearate

20 g of dried 12-oxime methyl stearate is added into 100 mL of 98% sulfuric acid, the raw material is prepared after drying according to the operation in embodiment 1, and the HPLC analysis shows that its purity is 99.2%. The mixture is slowly heated to 100°C, incubated for 1 hour, cooled to room temperature and poured into 200 g of broken ice, stirred and filtered to obtain 18 g of mixed amide ester. The subsequent operation follows embodiment 1 to obtain 8.9 g of 11-aminoundecanoic acid. The HPLC analysis shows that the purity of the product is 99.2%; 9.6 g of dodecanedioic acid. The HPLC analysis shows that the purity of the product is 99.4%.

The above embodiments are only for illustrating the technical idea and technical characteristics of the present invention, and are not intended to limit the protection scope of the present invention. All equivalent transformations or modifications made according to the essence of the present invention shall fall within the protection scope of the present invention.

## Claims

1. A method of co-producing a long-chain amino acid and a dibasic acid, **characterized by** comprising the following steps:
(1) performing an oximation reaction or an ammoxidation oximation reaction on a keto acid derivative (I) of the following formula 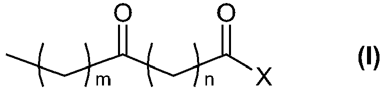 and hydroxyl amine in a solvent to generate a oximino acid derivative (II); 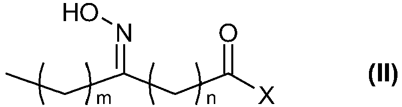 wherein in the above formulas, X is OR or NR1R2; OR is a monohydric alcohol or polyhydric alcohol of C1-C8, such as ethylene glycol, propylene glycol, butanediol or glycerin, etc.; R1 and R2 are respectively hydrogen and C1-C8 alkyl; m is an integer from 0 to 10; n is an integer from 6 to 20;
(2) performing a Beckmann rearrangement reaction on the dehydrated and dried oximino acid derivative in a solvent to generate a mixed amide derivative of formula (IIIa) and formula (IIIb) below; 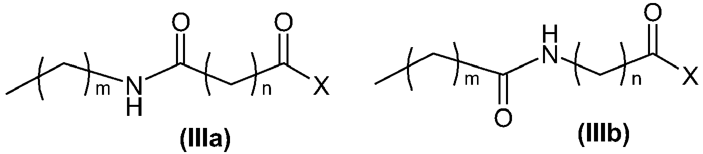
(3) hydrolyzing the above mixed amide derivative into a long-chain amino acid (V) and a dibasic acid (IV)of the following formulas.

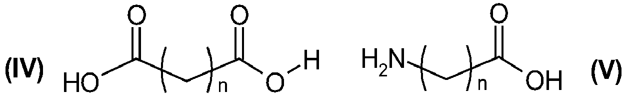

2. The method of co-producing a long-chain amino acid and a long-chain dibasic acid according to claim 1, the temperature of the oximation reaction is maintained between 0°C and 100°C, and the pH value is between 3 and 14.

3. The method of co-producing a long-chain amino acid and a long-chain dibasic acid according to claim 1, the catalyst for the Beckmann rearrangement reaction is sulfuric acid, or an compound containing acidic active halogen, or a mixture of Lewis acid and compound containing the acidic active halogen.

4. The method of co-producing a long-chain amino acid and a long-chain dibasic acid according to claim 1, the solvents used for the oximation reaction and the Beckmann rearrangement reaction may be different solvents or the same solvent.

5. The method of co-producing a long-chain amino acid and a long-chain dibasic acid according to claim 1, the product of the Beckmann rearrangement reaction is a mixed amide derivative (IIIa) and (IIIb).

6. The method of co-producing a long-chain amino acid and a long-chain dibasic acid according to claim 1, the mixed amide derivative is hydrolyzed to generate a long-chain amino acid (V) and a dibasic acid (IV).

7. The method of co-producing a long-chain amino acid and a long-chain dibasic acid according to claim 1, the mixed amide derivative is hydrolyzed with an alkali to generate a long-chain amino acid (V) and a dibasic acid (IV).

8. The method of co-producing a long-chain amino acid and a long-chain dibasic acid according to claim 1, the long-chain amino acid and the dibasic acid are separated and purified from the hydrolysis mixture by a stepwise neutralization method.

9. The method of co-producing a long-chain amino acid and a long-chain dibasic acid according to claim 1, the long-chain amino acid may be used as a monomer for producing long-chain nylon.

10. The method of co-producing a long-chain amino acid and a long-chain dibasic acid according to claim 1, the long-chain dibasic acid may be used as a monomer for producing long-chain nylon.
